# EUROPÄISCHE PATENTSCHRIFT

(11) **EP 1 490 832 B1**
(45) Veröffentlichungstag und Bekanntmachung des Hinweises auf die Patenterteilung: **02.11.2006**
(21) Anmeldenummer: 03744346.2
(22) Anmeldetag: 10.03.2003
(51) Int. Cl.: G06T 3/00

(54) **VIRTUELLES MIKROSKOP - VORRICHTUNG UND VERFAHREN**
VIRTUAL MICROSCOPE DEVICE AND METHOD
SYSTEME DE MICROSCOPE VIRTUEL ET PROCEDE ASSOCIE

(30) Priorität: 18.03.2002 DE 10213069; 31.05.2002 DE 10225174
(43) Veröffentlichungstag der Anmeldung: 29.12.2004
(73) Patentinhaber: Charité-Universitätsmedizin Berlin, 10117 Berlin (DE)
(72) Erfinder: SAEGER, Kai, 10557 Berlin (DE); SCHLÜNS, Karsten, 14050 Berlin (DE); HUFNAGL, Peter, 10318 Berlin (DE); DIETEL, Manfred, 14193 Berlin (DE)
(74) Vertreter: Schneider, Henry
(86) Internationale Anmeldenummer: PCT/EP2003/002443
(87) Internationale Veröffentlichungsnummer: WO 2003/079293

(56) Entgegenhaltungen:
- US-B1- 6 272 235
- FERREIRA R ET AL: "The Virtual Microscope" 1997 AMIA ANNUAL FALL SYMPOSIUM. A CONFERENCE OF THE AMERICAN MEDICAL INFORMATICS ASSOCIATION. PROCEEDINGS, PROCEEDINGS OF 1997 AMIA ANNUAL FALL SYMPOSIUM THE EMERGENCE OF INTERNETABLE HEALTH CARE SYSTEMS THAT REALLY WORK, NASHVILLE, TN, USA, 25-29 O, Seiten 449-453, XP002246194 1997, Philadelphia, PA, USA, Hanley & Belfus, USA
- LEONG F J ET AL: "Automated complete slide digitization: a medium for simultaneous viewing by multiple pathologists." THE JOURNAL OF PATHOLOGY. ENGLAND NOV 2001, Bd. 195, Nr. 4, November 2001 (2001-11), Seiten 508-514, XP002246195 ISSN: 0022-3417
- STRAUSS J S ET AL: "Virtual microscopy and public-key cryptography for Internet telepathology" JOURNAL OF TELEMEDICINE AND TELECARE, 1999, R. SOC. MED. PRESS LTD, UK, Bd. 5, Nr. 2, Seiten 105-110, XP002246196 ISSN: 1357-633X
- AFEWORK A ET AL: "Digital dynamic telepathology--the Virtual Microscope." PROCEEDINGS / AMIA... ANNUAL SYMPOSIUM. AMIA SYMPOSIUM. UNITED STATES 1998, 1998, Seiten 912-916, XP002246197 ISSN: 1531-605X
- WANG J Z ET AL: "Multiresolution browsing of pathology images using wavelets." PROCEEDINGS / AMIA... ANNUAL SYMPOSIUM. AMIA SYMPOSIUM. UNITED STATES 1999, 1999, Seiten 430-434, XP002246198 ISSN: 1531-605X
- SKODRAS A.; CHRISTOPOULOS C.; EBRAHIMI T.: 'The JPEG 2000 still image compression standard' IEEE SIGNAL PROCESSING MAGAZINE September 2001, Seiten 36 - 58
- ANONYMOUS: 'Using and distributing ECW V2.0 wavelet compressed imagery' COMPRESSION WHITE PAPER - VERSION 2.0, EARTH RESOURCE MAPPING PTY LTD., [Online] 31 Mai 1999, Gefunden im Internet: <URL:http://www.ermapper.com/document/doc.a spx?doc_id=72> [gefunden am 2005-12-15]

## Beschreibung

Die Erfindung betrifft ein Virtuelles-Mikroskop-System von einem Bildaufnahmesystem in einer vorgebbaren Vergrößerung und in einem vorgebbaren Ausschnitt gescannten oder anderweitig importierten digitalen Bilddaten eines Präparates, einem Serversystem mit einer Software zur Erzeugung eines Virtuellen Schnittes des Präparates aus den von dem Bildaufnahmesystem bereitgestellten oder anderweitig importierten digitalen Bilddaten des Präparates, einer Software zur Bildbearbeitung des Virtuellen Schnittes und einer Speichereinrichtung zur Speicherung des Virtuellen Schnittes in einer Falldatenbank, und mindestens einem Clientsystem mit einer Anwendersoftware zur Darstellung von durch einen Anwender auswählbaren Daten eines auswählbaren Virtuellen Schnittes der Falldatenbank. Die Erfindung betrifft ferner ein Verfahren zur Verarbeitung digitaler Mikroskopdaten nach Anspruch 35.

Zur Begutachtung von Objekten, wie zum Beispiel histologischen Präparaten, Schaltungen auf Prozessoren, oder anderen medizinischen Präparaten oder technischen Bauteilen unter starker Vergrößerung werden üblicherweise Lichtmikroskope benutzt. Lichtmikroskope lassen sich weiter unterteilen nach der Art der Präparatbeleuchtung (Auflicht- bzw. Durchlichtmikroskope), nach der Wellenlänge des Beleuchtungslichtes (einfaches Licht- bzw. Fluoreszenzmikrosköp), nach der Schwingungsrichtung des Beleuchtungslichtes (Polarisations- bzw. Interferenzkontrastmikroskop) sowie nach der Phasenlage des detektierten Lichtes (Phasenkontrast- bzw. Dunkelfeldmikroskop). Alle Lichtmikroskope sind in ihrer Auflösung durch die Wellenlänge des Lichtes beschränkt, und haben daher einen minimal unterscheidbaren Punktabstand von ca. 0,2 Mikrometer (0,2 x 10⁻⁶ m). Dies entspricht unter Berücksichtigung des vom menschlichen Auge minimal zu erkennenden Punktabstandes von ca. 0,2 Millimeter einer maximal möglichen Vergrößerung von etwa 1.000fach. Höhere Auflösungen werden mit Elektronenmikroskopen erzielt. Bei diesen wird das Präparat statt mit Lichtwellen mit den wesentlich kurzwelligeren Elektronen beschossen, und deren Ablenkung beim Durchfliegen des Präparates detektiert. Dadurch kann theoretisch eine Auflösung entsprechend einem Punktabstand von ca. 2 Angström (2 x 10⁻¹⁰ m) erreicht werden. Bedingt durch eine begrenzt erreichbare Linsenqualität ist tatsächlich allerdings nur eine sichtbare Vergrößerung von ca. 300.000fach möglich. Elektronenmikroskope werden nach der Art des Strahlengangs unterschieden (Transmissions- bzw. Rasterelektronenmikroskop).

All diese Geräte sind folgenden Einschränkungen unterworfen:
- Es kann immer nur ein Ausschnitt betrachtet werden.
- Es stehen nur wenige, bestimmte objektivabhängige Vergrößerungsstufen zur Verfügung.
- Es kann immer nur ein Präparat zur gleichen Zeit betrachtet werden.
- Es gibt keine Möglichkeit der Markierung auf lokaler mikroskopischer Ebene.
- Es gibt keine Möglichkeit einer inhaltsbezogenen Präsentation.
- Es kann nur einen lokalen Betrachter zur gleichen Zeit geben.
- Ein Präparat ist immer nur einmal vorhanden und kann nicht vervielfältigt werden.

Zudem ist die Möglichkeit der Kommentierung eines Falles stark eingeschränkt. Hinweise oder Bemerkungen zu dem Bild werden üblicherweise schriftlich notiert oder in ein Diktiergerät gesprochen. Die Verknüpfung von Hinweisen mit konkreten Orten auf dem Präparat ist ohne Hilfsmittel (z.B. Scanningtisch und Software) nicht eindeutig möglich. Die interessierenden Orte muss sich der Anwender im Allgemeinen merken. Die Begutachtung eines Objektes in räumlicher Entfernung vom Mikroskop ist ohne spezielle Hilfsmittel (wie z.B. einer TV-Kamera) nicht möglich, ebenso das Versenden eines Falles in digitaler Form.

Zur Verbesserung der Funktionalität solcher Mikroskope wurden in der Vergangenheit diverse Zusatzkomponenten entwickelt, die jeweils einzelne technische Beschränkungen eines Mikroskops lösen. So ermöglicht eine so genannte "Mehrfachsichteinrichtung" mehreren Personen die gleichzeitige Betrachtung eines Präparates durch eine am Mikroskop anzubringende strahlenteilende Optik. Das schmale Sichtfeld kann durch eine spezielle Okularoptik (Großfeldoptik) erweitert werden. Videokameras mit Monitor gestatten mehreren Personen die gleichzeitige Betrachtung des Präparats, allerdings in stark eingeschränkter Qualität. Die Erzeugung digitaler Bilder eines einzelnen Ausschnitts und deren Vervielfältigung ist ebenfalls mittels Videokamera und Computeranschluss realisiert worden. Und schließlich existieren seit ein paar Jahren vermehrt Telepathologiesysteme, mit denen die Live-Übertragung des Mikroskopbildes an einen entfernten Anwender sowie die Femsteuerung des Mikroskops über diesen entfernten Anwender ermöglicht wurde.

In jüngster Zeit sind auch digitale Verfahren zur Unterstützung von Mikroskopen entstanden. So ist zum Beispiel aus der WO 98/39728 das Erstellen digitalisierter Mikroskopbilder, so genannter "Virtueller Schnitte" (VS), über rechnergesteuerte Mikroskope bekannt. Dabei wird ein aus einer Vielzahl von Teilbildern zusammengesetztes Übersichtsbild geringer Auflösung aufgenommen und die Teilbilder mit einer gemeinsamen, Koordinaten-gebundenen Datenstruktur verknüpft und gespeichert. Der Anwender kann nachfolgend interessierende Bereiche auswählen, in denen die Daten in einer bestimmten höheren Auflösung bzw. Vergrößerungsstufe gemessen und ebenfalls Koordinaten-gebunden gespeichert werden. Die in dieser Schrift beschriebene Datenstruktur sieht nicht die Bereitstellung eines Gesamtbildes des Präparates in höherer Auflösung vor. Ebenso ist nicht die Möglichkeit zur Erstellung von Multilayer-Bildern sowie der Anwendung einer Zeichnungsebene oder stufenloses Zoomen durch den Anwender realisiert.

Aus der WO 01/54052 ist bekannt, die Daten eines Virtuellen Schnittes auf einem Server zu speichern und clientseitig mittels eines Webbrowsers in Form eines Übersichtsbildes niedriger Auflösung und abrufbarer Teilbilder höherer Auflösung zu visualisieren, wobei ein Hin- und Herschalten zwischen den verschiedenen Auflösungen vorgesehen ist. Auch hier wird nicht ein Gesamtbild in höchster Auflösung bereitgestellt. Zudem ist nicht die Möglichkeit der Nutzung von Multilayer-Bildern und einer Zeichnungsebene vorgesehen. Auch findet kein stufenloses Zoomen statt, und keine Verwaltung der Benutzerdaten und Falldaten.

Die WO 99/47964 beschreibt spezielle Techniken zum Scannen eines Präparats.

Schließlich ist aus der WO 01/26541 bekannt, die Virtuellen Schnitte als Datenstruktur über Inter- und Intranet zu verschicken.

Die Druckschrift Ferreira et al. ("The Virtual Microscope", 1997, AMIA Aminal Fall Symposium - A Conference of the American Medical Informatics Association, Proceedings of 1997, Nashville, USA, S. 449-453) beschreibt ein virtuelles Mikroskop, bei dem Bilddaten eines Präparates vor ihrer Kompression mit einem wavelet-basierten Verfahren und ihrer Speicherung in Blöcke unterteilt werden, die nach jeweils individueller Kompression auf unterschiedliche Speicherplätze eines parallelen Datenservers verteil gespeichert werden.

Aus der Telepathologie ist zudem die Möglichkeit der Live-Diskussion eines Falls durch mehrere Anwender über das Internet bekannt.

Zusammenfassend ist festzustellen, dass noch kein System zur Verfügung steht, welches (abgesehen von der Bildaufnahme, dem Scannen) ein Mikroskop tatsächlich in Gänze ersetzen könnte. Solch ein System wäre wünschenswert, da es alle Vorteile in einer Lösung abdeckt, billiger und komfortabler wäre und zudem Funktionalitäten ermöglicht, die von Mikroskopanwendern dringend gewünscht wären. Zu solchen Funktionalitäten gehören beispielsweise die Anwendung von Multilayer-Bildern, die Nutzung einer Markierungsebene, die automatische Verwaltung von Fällen zur Automatisierung eines Bearbeitungsablaufs (Workflows), die automatische Bildbearbeitung der Bilder zur Unterstützung und Beschleunigung der Arbeit eines Mikroskopanwenders sowie das stufenlose Zoomen.

Der Erfindung liegt somit die Aufgabe zugrunde, ein so genanntes Virtuelles Mikroskop zur Verfügung zu stellen, welches mit Ausnahme der eigentlichen Bildaufnahme alle Funktionalitäten eines herkömmlichen mechanischen Licht- oder Elektronenmikroskops vollständig ersetzt und zudem einige zusätzliche, von der mechanischen Lösung bisher unbekannte Funktionalitäten bietet. Ferner soll das Virtuelle Mikroskop die genannten Beschränkungen heutiger digitaler Mikroskope überwinden und insbesondere eine Fernübermittlung der Bilddaten in Echtzeit ermöglichen. Es soll außerdem ein entsprechendes Verfahren zur Verarbeitung digitaler Mikroskopdaten bereitgestellt werden.

Die Aufgabe wird durch ein Virtuelles Mikroskop mit den Merkmalen des Anspruchs 1 und ein Verfahren nach Anspruch 35 gelöst. Das erfindungsgemäße Virtuelle Mikroskop ist dadurch gekennzeichnet, dass die von dem Bildaufnahmesystem, beispielsweise einem Licht- oder Elektronenmikroskop, übermittelten, oder anderweitig (beispielsweise aus einem PACS) importierten, digitalen Bilddaten des Präparates und der serverseitig aus diesen erzeugte Virtuelle Schnitt ein Bild des Präparates in der höchsten verfügbaren oder gewünschten Vergrößerung und dem höchsten verfügbaren oder gewünschten Ausschnitt des Präparates, insbesondere eine Gesamtansicht des Präparates sind.

Dieser Virtuelle Schnitt wird in einer Datei abgelegt, auf den clientseitig stets zugegriffen wird. Erfindungsgemäß umfasst der Virtuelle Schnitt ein Multilayer-Bild aus mehreren, einander überlagernden Bildlayern desselben Präparates, die durch automatisches Matching auf einen gemeinsamen geometrischen Bezug gebracht sind.

Demnach beinhaltet die Datenbank erfindungsgemäß lediglich jeweils ein digitales Mikroskopbild eines Präparates in einer einzigen, nämlich der maximalen Vergrößerung. Dabei bedeutet hohe oder niedrige Vergrößerung eine Darstellung seitens des Anwenders in hoher bzw. niedriger Auflösung, wie in der Darstellung digitaler Bilder auf Monitoren üblich. Auf diese Weise kann ein Anwender an einer Clientstation stets unmittelbar auf die Virtuellen Schnitte zugreifen, ohne dass beispielsweise erneute mikroskopische Messungen in dem interessierenden Bereich mit der gewünschten Vergrößerung durchgeführt werden müssen.

Das Grundprinzip der Erfindung ist also das Scannen eines ganzen Präparates unter der höchsten verfügbaren bzw. notwendigen Vergrößerung zu einem Gesamtbild, dessen Darstellung in einer Internet- oder Intranet-basierten oder unabhängigen Oberfläche und die Bearbeitung des Falles unter Verwendung neuer digitaler Werkzeuge in einer Client-Server-Umgebung. Der komplette Objektträger wird in höchster Vergrößerung digitalisiert und als elektronische Datei (Virtueller Schnitt) auf einem Rechner (Server) abgelegt. Das Bild kann von einer an einem Mikroskop (gegebenenfalls fernsteuerbar) installierten Digitalkamera von importierten Digitalbildern, einem sonstigen Bildaufnahmesystem oder einem PACS (Picture Archiving and Communications System) stammen. Die Bilddaten liegen dabei vorzugsweise in komprimierter Form vor, da sehr große Datenmengen zu bewältigen sind. Das Bild kann über Inter- oder Intranetprotokolle (wie TCP/IP) oder über feste Datenträger (wie zum

Beispiel einer CD-ROM) in das System geladen werden. Zur Betrachtung der Virtuellen Schnitte (VS) dient nun das Virtuelle Mikroskop-System, welches genau den gewünschten Präparatausschnitt oder auch das gesamte Präparat auf einen Computermonitor eines Clientsystems visualisiert. Die Steuerung erfolgt über ein interaktives Eingabemedium (etwa Maus, Joystick, Touchpad, ...) durch den Anwender.

Die Umsetzung der Begutachtung von Objekten unter starker Vergrößerung in digitaler Form bringt erhebliche zeitliche und funktionale Verbesserungen mit sich. Ist ein Objekt einmal mittels eines geeigneten Bildaufnahmesystems digitalisiert (beispielsweise mit einem herkömmlichen Mikroskop mit Scanningtisch und Digitalkamera), so kann es mit Hilfe des erfindungsgemäßen Systems über Intra- oder Internet jedem gewünschten Anwender zugänglich gemacht werden, auch mehreren gleichzeitig. Das digitale Bild kann mit einem zusätzlichen Layer (Markierungsebene) überlagert werden, auf dem Bemerkungen oder Hinweise angebracht, oder interessante Orte markiert werden können. Zudem ermöglicht die digitale Form stufenloses Zoomen und Verschieben sowie diverse Methoden der Bildverarbeitung.

Nach einer besonders vorteilhaften Ausgestaltung der Erfindung umfasst die Anwendersoftware des Clientsystems einen Viewer, in dem die Gesamtansicht des Präparates als Übersichtsbild mit geringer Auflösung und/oder mindestens ein durch den Anwender aktuell ausgewählter Bildbereich als Detailansicht in höherer Auflösung dargestellt wird. Vorzugsweise wird neben der Darstellung des ausgewählten Präparatausschnittes ständig das Übersichtsbild dargestellt, welches das gesamte Präparat und vorzugsweise darauf markiert den Bereich des gewählten Ausschnittes anzeigt. Zusätzlich können auch mehrere Ausschnitte gleichzeitig betrachtet werden. Sowohl im Übersichtsbild als auch in allen Detailansichten kann die Auflösung durch den Anwender frei eingestellt werden.

Es sind ferner vorteilhaft Mittel innerhalb des Clientsystems vorgesehen, mit denen genau ein durch den Anwender ausgewählter Bildbereich des ausgewählten Virtuellen Schnittes von der Falldatenbank des Serversystems jeweils aktuell angefordert und clientseitig in dem Viewer dargestellt wird. Wahlweise können die Daten auch über mobile Datenträger (beispielsweise CD-ROM) im Client-Server-System geladen werden. Die durch die Software zur Erzeugung Virtueller Schnitte erzeugten hochkomprimierten Bilddaten, die vorzugsweise im so genannten Enhanced-Compressed-Wavelet-Format (ECW, Format der Firma Earth Ressource Mapping) vorliegen, erlauben dabei die Übermittlung und Darstellung des ausgewählten Bereichs quasi in Echtzeit. Dabei werden jeweils ausschließlich die Daten des interessierenden Bildbereichs in der Kompressionsstufe der interessierenden Auflösung vom Serversystem an das Clientsystems übermittelt, clientseitig mittels einer Dekomprimierungssoftware, vorzugsweise in Form eines dem Komprimierungsformat entsprechenden Plug-Ins der Firma "Earth Ressource Mapping", dekomprimiert und in dem Viewer in der gewünschten Größe dargestellt.

Da erfindungsgemäß das Gesamtbild des Präparates in höchster Auflösung in Form des Virtuellen Schnittes vorliegt, wird eine stufenlose digitale Zoomfunktion realisiert, die eine Detailansicht innerhalb jeden beliebigen Ortes innerhalb der Gesamtansicht des Virtuellen Schnittes bis zur höchsten Vergrößerung (bzw. Auflösung) gestattet. Dafür setzt der Anwender manuell beispielsweise einen in der Gesamtansicht oder der Detailansicht vorgesehenen Tracker, um die Position und Ausdehnung des gewünschten Teilbereichs festzulegen, woraufhin das Clientsystem die erforderlichen Bilddaten vom Serversystem anfordert und als neue Detailansicht darstellt. Dabei entspricht - wie beim digitalen Zoomen üblich - die Darstellung eines Ausschnittes oder Bildes in niedriger Vergrößerung einfach der Darstellung des Bildes in niedriger Auflösung. Damit kann ein Präparat stufenlos in jeder beliebigen Vergrößerungsstufe angezeigt werden. Das Zoomen wird in Echtzeit vollzogen, so dass der Anwender interaktiv die optimale Vergrößerungsstufe einstellen kann. Der Tracker kann durch manuelles Verschieben auch zum Durchrastern des Virtuellen Schnittes eingesetzt werden.

Einen besonderen Vorteil der Erfindung stellt die Möglichkeit der Nutzung von Multilayer-Bildern dar, die aus mehreren einander überlagernden Gesamtbildern desselben Präparates - gemessen etwa unter Verwendung verschiedener Färbungen oder Aufnahmetechniken - bestehen. Multilayer-Bilder sind im Prinzip bekannt. Es handelt sich dabei um einen Bilddatensatz, der innerhalb einer Datei mehrere Bilder desselben Objektes enthält, die geometrisch aufeinander angepasst sind. Dies ermöglicht dem VM-Anwender die tatsächlich gleichzeitige Betrachtung und somit den direkten und schnellen visuellen Vergleich zweier oder mehrerer Bilder eines Objektes, wie es mit heutigen Mikroskopen und auch den bisherigen Digitallösungen bezogen auf das Gesamtbild des Präparates nicht möglich war. Die zeitgleiche Visualisierung der Bilder erfolgt über Teilung der Detailansicht, einfaches und schnelles Umschalten der Ansicht oder auch über eine halbtransparente Sicht auf mehrere Präparate mit der gleichzeitigen Möglichkeit der Einstellung der Transparenz. Die geometrische Anpassung der Bilder (Matching) aufeinander erfolgt innerhalb eines Bildbearbeitungsmoduls auf dem Server.

Hierbei ist insbesondere auch bevorzugt möglich, mehrere Detailansichten gleichzeitig darzustellen, wobei insbesondere die Detailansichten in verschiedener Vergrößerung und/oder verschiedener Färbung und/oder an verschiedenen Orten und/oder in verschiedenen Zoomdarstellungen darstellbar sind.

Ein weiterer entscheidender Vorteil der Erfindung ist, dass das Bild bzw. Multilayer-Bild mit einer Markierungsebene überlagerbar ist, auf der durch den Anwender Bildkoordinaten-bezogene Kommentare, Markierungen und/oder dergleichen untergebracht werden können. Dies ermöglicht die Markierung interessierender Orte oder Bereiche, das Eintragen von Bildkoordinaten-bezogenen Texten, Symbolen, Grafiken, Zeichnungen, Markierungen, Hyperlinks, Verweisen auf andere Daten (z.B. Audio-Daten) und das Zeichnen, auch freihändig, im Bild, sowie die Verknüpfbarkeit von markierten Orten und weiteren Präparatinformationen nach Bedarf. Ferner kann die Markierungsebene Markierungen der während der Bildanalyse betrachteten Bildbereiche und/oder eines so genannten Inspektionspfades umfassen, der einen von dem Anwender inspizierten Weg durch das Präparat darstellt. Die Markierungsebene kann wahlweise angezeigt werden oder unsichtbar vorgesehen sein. Es ist ferner möglich, die Markierungsebene zusammen mit dem Virtuellen Schnitt in der Falldatenbank dauerhaft zu speichern.

Gemäß einer bevorzugten Ausgestaltung der Erfindung umfasst die Software zur Erzeugung Virtueller Schnitte, die auf dem Serversystem installiert ist, eine Importfunktion, die den Import der vom Aufnahmesystem bereitgestellten Bilddaten in beliebigen Bilddatenformaten erlaubt. Die Bilddatenformate werden dann durch die Software in das komprimierte Bildformat (insbesondere in ECW-Format) konvertiert wobei eine Farbtiefe variabel festgelegt werden kann. In dieser Form werden die Bilddaten eines Virtuellen Schnittes gespeichert. Neben den eigentlichen Bilddaten enthalten die in der Falldatenbank gespeicherten Virtuellen Schnitte weitere Informationen, insbesondere Statusdaten des Falles, Präparations- und Aufnahmeparameter und/oder dergleichen, wobei diese Textdaten vorzugsweise SQL-strukturiert (Structured Query Language, ein vielfältiges Abfrageformat für Datenbanken) vorliegen. Die Textdaten können von einem Bediener des Aufnahmesystems und/oder clientseitig von einem Anwender mit üblichen Eingabemitteln eingegeben werden.

Eine weitere besonders vorteilhafte Ausgestaltung der Erfindung sieht serverseitig die Bereitstellung einer Software bzw. eines Softwarepaketes zur Bildbearbeitung der Virtuellen Schnitte vor, die vom Serversystem nach Erzeugung des Virtuellen Schnittes automatisch - entsprechend dem Falltyp - gestartet oder individuell durch einen Anwender ausgeführt werden kann, wobei die Bildbearbeitung einen oder mehrere der Schritte
- Kontrastverbesserung
- Normierung der Helligkeitsverteilung
- Weißabgleich
- Kantenextraktion
- Matching auf bekannte Strukturen zur Vorauswahl interessierender Bereiche
- Messen und Zählen von Strukturen
und/oder weitere Funktionen umfasst. Derartige Bildbearbeitungsfunktionen können nach weiteren bevorzugten Ausgestaltungen der Erfindung insbesondere auch eine Simulation von Beleuchtungssituationen, insbesondere eine Simulation wenigstens eines Hellfeldes und/oder Dunkelfeldes und/oder Polarisation und/oder Auflicht und/oder Durchlicht und/oder Phasenkontrast oder dergleichen, sein. Ferner kann die Bildbearbeitungsfunktion eine Simulation von Farbsituationen, insbesondere Erzeugung von Mischfärbungen als Kombination von realen Färbungen und/oder Farbwechsel sein. Die Bildbearbeitungsschritte können entweder von einem Anwender aufgerufen werden, oder entsprechend bestimmter Falltypen automatisch gestartet werden. Es kann eine Liste von Bildbearbeitungsschritten für relevante Falltypen angelegt werden, so dass das System entsprechend dem aktuellen Falltyp automatisch festgelegte Schritte startet. Die Bildbearbeitung dient dazu, automatisch Strukturen hervorzuheben, die den Anwender bei der späteren Beurteilung des Bildes unterstützen bzw. ihn gezielt auf relevante Strukturen (z.B. Mitosen in Tumorpräparaten) hinweisen.

Nach einer weiteren vorteilhaften Ausgestaltung der Erfindung sind Mittel zur Verwaltung der Fall-, Bild- und Anwenderdaten vorgesehen. Dabei existiert neben der Falldatenbank, in der alle Falldaten, Bilddaten und Statusdaten eines Falles strukturiert (beispielsweise mittels SQL) abgelegt sind, eine Benutzerdatenbank, in der Zugangsdaten, Zugriffsrechte und Zuordnungen zu Fällen gespeichert sind. Das Serversystem, auf dem diese Datenbanken abgelegt sind, sorgt dabei für eine sichere, redundante Speicherung der Daten (beispielsweise mittels RAID-System). Erst die sorgfältige Ausgestaltung dieser beiden Datenbanken ermöglicht es, mit dem Virtuellen-Mikroskop-System ein herkömmliches Mikroskop in Gänze zu ersetzen. Dazu gehört auch eine detaillierte Protokollierung des Workflows und der Arbeit des Anwenders.

In weiterer bevorzugter Ausgestaltung der Erfindung ist vorgesehen, dass die Zugangssteuerung von Anwendern auf das Serversystem Mittel zur Verschlüsselung/Entschlüsselung und/oder Mittel zur Prüfung der Zugangsberechtigung, insbesondere als digitale Signatur, umfasst. Hierdurch wird eine sichere Authentifizierung beim Zugriff von Benutzern möglich.

Das erfindungsgemäße System umfasst ferner einen Konferenzmodus zwischen den Clientsystemen, wobei insbesondere zwischen dem Clientsystem und/oder dem Clientsystem und dem Serversystem ein Steuerungswechsel, insbesondere eine Master-Slave-Zuordnung möglich ist. Hierdurch werden vorteilhafterweise zwischen den Clientsystemen und/oder dem Clientsystem und dem Serversystem Live-Diskussionen, insbesondere audio-visuelle Live-Diskussionen, möglich.

Das erfindungsgemäße Verfahren zur Verarbeitung digitaler Mikroskopdaten, umfasst die Schritte:
a. Scannen oder anderweitiger Import digitaler Bilddaten eines Präparates in einer höchsten verfügbaren oder gewünschten Vergrößerung und einem höchsten verfügbaren oder gewünschten Ausschnitt des Präparates durch ein Bildaufnahmesystem,
b. Erzeugung eines Virtuellen Schnittes in der höchsten verfügbaren oder gewünschten Vergrößerung und dem höchsten verfügbaren oder gewünschten Ausschnitt des Präparates aus den von dem Bildaufnahmesystem bereitgestellten oder anderweitig importierten digitalen Bilddaten des Präparates mittels einer ersten Software eines Serversystems,
c. Veränderung des Virtuellen Schnittes mittels Bildbearbeitungsfunktionen einer zweiten Software des Serversystems,
d. Speichern des Virtuellen Schnittes in einer Falldatenbank einer Speichereinrichtung und
e. Darstellung von durch einen Anwender mindestens eines Clientsystems ausgewählten Daten eines ausgewählten Virtuellen Schnittes der Falldatenbank mittels einer Anwendersoftware.

Das Verfahren ist dadurch gekennzeichnet, dass alle Daten eines Präparates als ein Virtueller Schnitt in einer Datei abgelegt werden und clientseitig stets auf diese Datei zugegriffen wird. Dieser Virtuelle Schnitt umfasst ein Multilayer-Bild aus mehreren, einander überlagernden Bildlayern desselben Präparates, die durch automatisches Matching auf einen gemeinsamen geometrischen Bezug gebracht sind.

Weitere vorteilhafte Ausgestaltungen der Erfindung sind Gegenstand der übrigen Unteransprüche.

Die Erfindung wird nachfolgend anhand der zugehörigen Zeichnungen näher erläutert. Es zeigen:
- Figur 1: schematisch den Datenfluss des Gesamtsystems eines Virtuellen Mikroskops;
- Figur 2: schematisch die Funktionsweise des Serversystems innerhalb des Gesamtsystems, und
- Figur 3: schematisch die Funktionsweise eines Clientsystems innerhalb des Gesamtsystems.

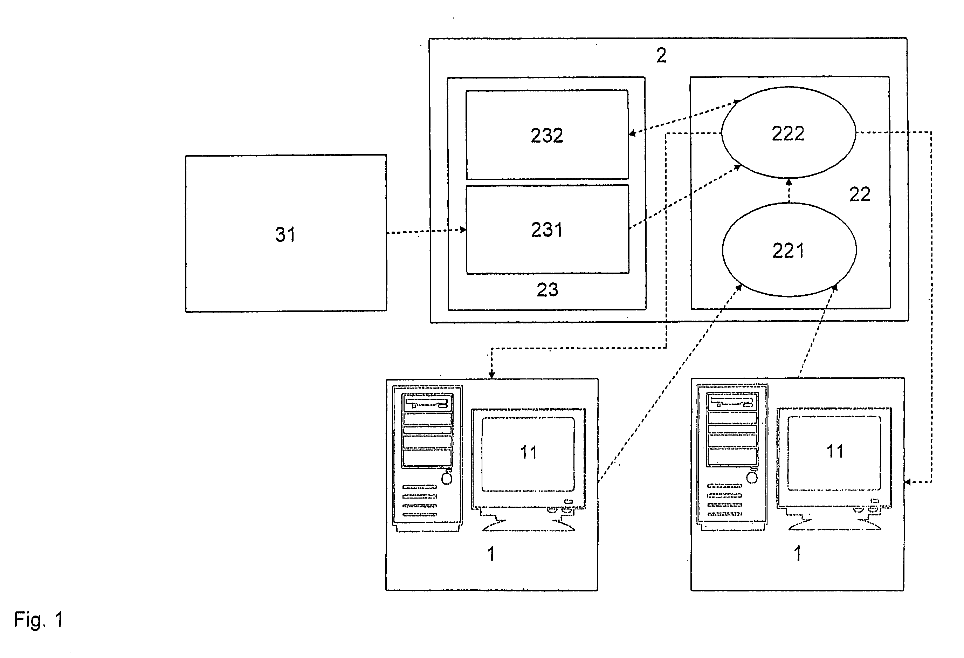

Gemäß Figur 1 handelt es sich bei dem erfindungsgemäßen Virtuellen Mikroskop um ein Client-Server-basiertes System. Es umfasst die zwei Hauptkomponenten, bestehend aus mindestens einem Clientsystem 1 und einem Serversystem 2, wobei allerdings auch ein Client 1 zusammen mit dem Serversystem 2 auf demselben Rechner installiert sein kann.

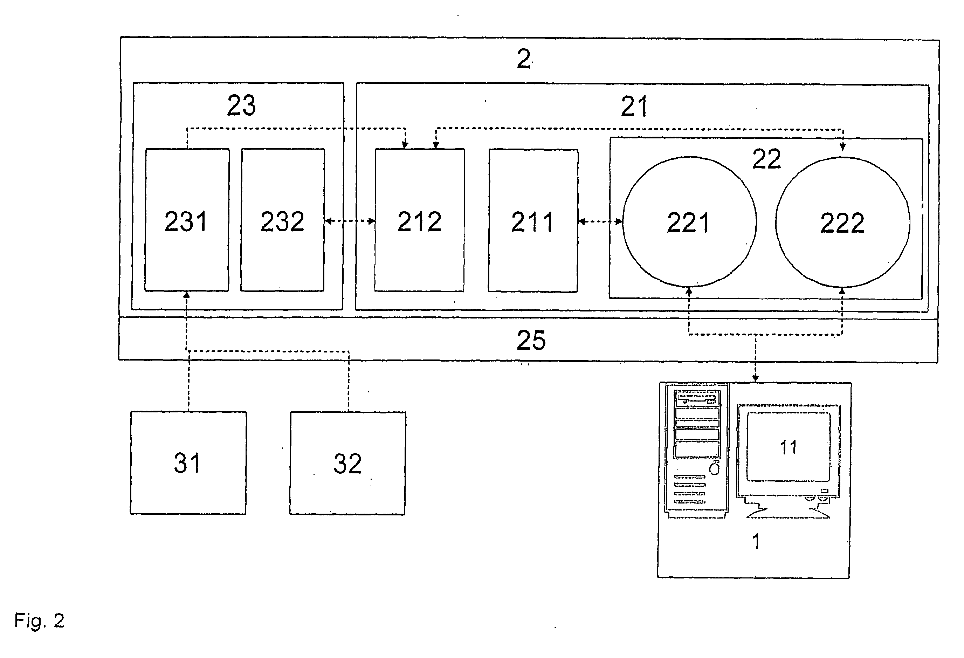

Das Serversystem 2 umfasst ein Speichersystem 21 (Figur 2), auf dem alle Virtuellen Schnitte 212 eines Falles abgelegt werden. Daneben stellt der Server 2 auch Serversoftware 23, insbesondere eine Software 231 zur Erzeugung Virtueller Schnitte 212 und eine Software 232 zur Bildbearbeitung der Virtuellen Schnitte 212 bereit.

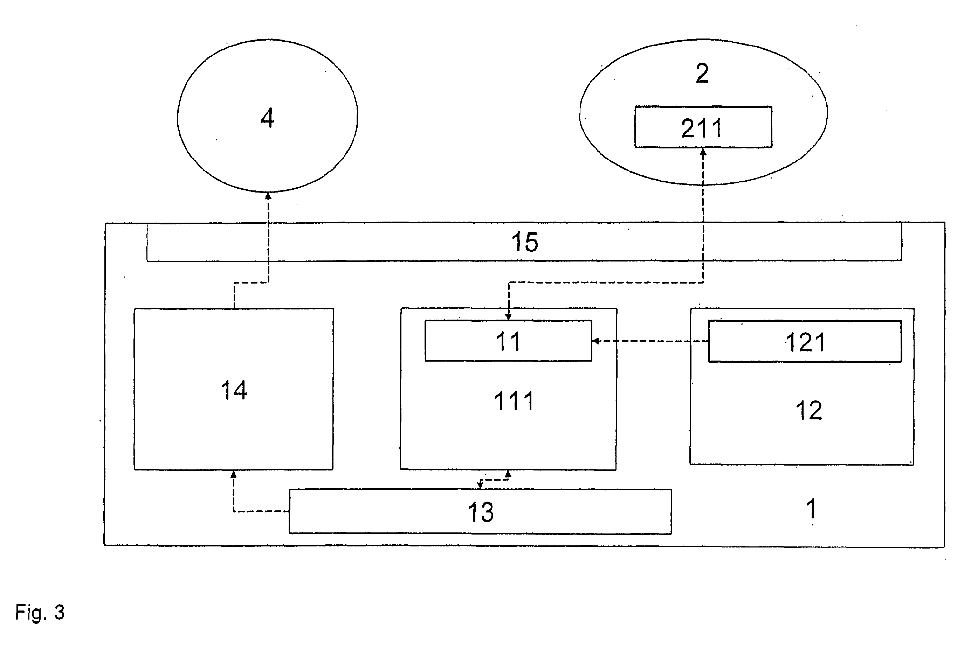

Als Clientsystem 1 gelten alle, über Inter- oder Intranet mit dem Serversystem 2 kommunizierenden Rechnerstationen, die von Anwendern 13 (Figur 3) des Systems bedient werden. Es können beliebig viele Clientsysteme 1 vorhanden sein, die auch gleichzeitig auf den Server 2 zugreifen können. Zur Kommunikation und Datenübertragung verfügt jedes Clientsystem 1 über eine Schnittstelle 15 und das Serversystem 2 über eine Schnittstelle 25, die vorzugsweise TCP/IP-Schnittstellen sind. Clientseitig wird zur Anforderung, Darstellung und Begutachtung eines Virtuellen Schnittes 212 eine Anwendersoftware 11 benötigt, die auf jedem Clientsystem 1 laufen kann. Die Anwendersoftware 11 wird gemäß dem dargestellten Beispiel vom Serversystem 2 innerhalb eines Webbrowsers 111 bereitgestellt. Optional kann die Anwendersoftware 11 jedoch auch als eigenständige Programmoberfläche oder innerhalb einer Oberfläche einer anderen Software im Clientsystem 1 bereitgestellt werden.

Nachfolgend wird auf einige Einzelkomponenten von Server- und Clientsystem näher eingegangen.

### Server

Das Serversystem 2 besteht aus dem Speichersystem 21, der insbesondere ein Festspeicher ist, und der ein Datenbankmodul 22 umfasst, und Software 23 und die Schnittstelle 25.

Das Datenbankmodul 22 hat folgende Aufgaben:
- Speicherung der von einem Bildaufnahmesystem aufgenommenen digitalen Bilder in Form von Virtuellen Schnitten 212 in einer Falldatenbank 222,
- Betrieb der Serversoftware 23, mit der die Bilder und Daten betrachtet und verändert werden können,
- Verwaltung der Bild- und Textdaten in den Datenbanken 22,
- Zugangssteuerung über eine Benutzer-Zugangs-Datenbank 221, welche die angemeldeten Benutzer 13 und deren Zugriffsrechte verwaltet.

Die Speicherung der Bilddaten erfolgt in einem speziellen Bilddatenformat, welches eine hohe Kompression ermöglicht (vorzugsweise in ECW-Format). Die beschreibenden Daten und Falldaten (212) werden SQL-basiert verwaltet. Dies ermöglicht das Finden bestimmter Fälle anhand aller erfassten Parameter.

Das Serversystem 2 beinhaltet weiterhin einige Softwarekomponenten 23. Dies sind:
1. Die Anwendersoftware 11, die an den Webbrowser 111 oder als eigenständiges Programm eines Anwenders 13 übertragen werden kann. Diese Anwendersoftware 11 dient als Anwenderoberfläche. Sie beinhaltet ein erstes Anzeigefenster, in dem ein Übersichtsbild des Virtuellen Schnitts 212 dargestellt wird und über den im Virtuellen Schnitt navigiert werden kann, ein weiteres Anzeigefenster, das einen aktuell ausgewählten Bildausschnitt anzeigt und mehrere Steuerungstools, die zur Navigation im Bild, zur Bearbeitung der Falldaten und zur Datenverwaltung dienen. Diese Anwendersoftware kann clientseitig alternativ auch von einem festen Datenträger (beispielsweise einer CD-ROM) geladen werden.
2. Ein Programm 231 zur Erzeugung der Virtuellen Schnitte 212. Das Programm ist nötig, um die digitalen Bilddaten 31 eines Bildaufnahmesystems in einen Virtuellen Schnitt 212 zu formatieren, der dann in der Falldatenbank 222 abgelegt wird. Initial besteht ein Virtueller Schnitt 212 aus den Bilddaten, wobei auch Multilayer-Daten möglich sind, und einigen Daten 32 wie zum Beispiel Fallnummern, Aufnahmedatum und ähnliches, die vom Anwender 13 interaktiv eingegeben bzw. über eine Schnittstelle aus einem anderen Datenbanksystem (z.B. Krankenhausinformationssystem oder PACS) importiert werden. Die Daten eines solchen Virtuellen Schnittes 212 wachsen aber bei der Bearbeitung des Falles an, da nachfolgend diverse Anmerkungen, Marker und ähnliches hinzukommen. Das Programm 231 zur Erzeugung der Virtuellen Schnitte 212 hat also die Aufgabe, die übertragenden Bilddaten in das spezielle Bildformat eines Virtuellen Schnittes 212 umzuformatieren, falls nötig Teilbilder zu einem Gesamtbild zusammenzufügen oder mehrere Bildlayer so zusammen zu matchen, dass sie einen gemeinsamen geometrischen, beispielsweise koordinatenbezogenen Bezug bekommen. Außerdem müssen die vom Anwender 13 angegebenen Daten in das Format des Virtuellen Schnittes 212 eingetragen werden. Der Ablauf der Virtuellen-Schnitt-Erzeugung ist wie folgt:
   - Zunächst werden digitale Bilddaten 31, die von dem Bildaufnahmesystem in raw, tiff, jpg oder einem anderem Format bereitgestellt werden, geladen.
   - Entsprechend dem aktuellen Falltyp (präparatabhängig) werden diverse Bildbearbeitungsschritte in einer festgelegten Sequenz angewandt.
   - Dabei erfolgt eine Farbanpassung auf eine festgelegte Farbtiefe (z.B. 16 Bit = 2x5 Bit + 1x6 Bit).
   - Nachfolgend wird in ein komprimiertes Bildformat (z.B. ECW) umformatiert.
   - Es erfolgt eine Überlagerung und ein geometrisches Matching zusätzlicher Bildlayer zur einem Multilayer-Bild.
   - Zusatzinformationen durch Interaktion mit dem Anwender 13 werden angebracht.
   - Fallnummer, Bearbeiter, Status, Falltyp werden erstellt.
   - Alle Bild- und Textdaten werden als ein Virtueller Schnitt 212 in der Falldatenbank 222 abgelegt.
3. Eine Software 232 zur Bildbearbeitung Virtueller Schnitte 212. Diese kann alle Methoden der Bildverarbeitung umfassen, die an einem Digitalbild anwendbar sind, um automatisch Strukturen hervorzuheben, die den Anwender 13 bei der späteren Beurteilung des Bildes unterstützen. Solche Methoden sind zum Beispiel:
   - Kontrastverbesserung,
   - Normierung der Helligkeitsverteilung,
   - Weißabgleich,
   - Kantenextraktion,
   - Matching auf bekannte Strukturen zur Vorauswahl interessanter Bereiche,
   - Erkennen, Messen und Zählen von Objekten und Strukturen,
   - diverse weitere.

   Die Bildbearbeitungsschritte können entweder von einem Anwender 13 aufgerufen werden, oder entsprechend bestimmter Falltypen automatisch gestartet werden. Es kann eine Liste von Bildbearbeitungsschritten für jeden benutzen Falltyp angelegt werden, so dass das System entsprechend des aktuellen Falltyps automatisch festgelegte Schritte startet.

Schließlich gehört zum Serversystem 2 auch eine Schnittstelle 25, mit der der Server 2 mit den Clientsystemen 1 verbunden werden kann. Dies wird vorzugsweise über TCP/IP realisiert.

### Client

Auf Anwenderseite 1 ist die Anwendersoftware 11 über einen Webbrowser 111 steuerbar oder läuft als eigenständige Oberfläche ab und ist dadurch systemunabhängig. Der Viewer für große Bilddateien wird auf dem anwenderseitigen Webbrowser 111 bzw. der eigenständigen Programmoberfläche angeboten. Die Bilddaten werden sämtlich serverseitig gespeichert. Dabei werden allein die interessierenden Bilddaten vom Server- zum Clientsystem transferiert und dekomprimiert. Das spezielle Format für die Bilddaten sorgt dabei für eine hohe Kompression ohne sichtbaren Qualitätsverlust bei schnellem Zugriff auf einzelne Bereiche innerhalb der Bilddatei. Eine hohe Kompressionsrate ist bei der enormen Größe der Bilddaten besonders wichtig. Der schnelle Zugriff auf die Bilddaten ermöglicht das Zoomen und Durchrastern der Bilder annähernd in Echtzeit. Der Zugang zur Software erfolgt über ein Login, um Zugriffsrechte zu steuern und die Daten zu schützen. Dadurch wird jedem Anwender nur der Zugriff auf bestimmte Daten gewährt, und er erhält für jeden Datentyp bestimmte Verwaltungsrechte (zum Beispiel Erzeugen, Lesen, Verändern, Löschen).

Das Anwenderprogramm 11 des Clientsystems 1 kann - neben dem erfindungsgemäß automatischen Matching - eine Reihe folgender Funktionalitäten beinhalten, wobei besonders bevorzugte Basisfunktionalitäten die Punkte 1a bis 1g, 2h, 3l, 4p sowie 7x bis 7z, vorteilhafte sinnvolle Hauptfunktionalitäten die Punkte 2i und 2j, 3m und 3n, 4q und 4r sowie 6v und optionale Funktionalitäten mit lediglich Zusatzfunktion die Punkte 2k, 3o, 4s, und 5u, 6w sowie 7aa darstellen:
1. Die Bildbewegung kann mit variablen Eingabegeräten ermöglicht werden, wobei folgende Funktionen vorgesehen sind:
   a. stufenloses Bewegen des Bildes (interaktiv),
   b. stufenloses Zoomen des Bildes (interaktiv),
   c. Verschieben des Bildes um einen bestimmten Betrag (halbautomatisch),
   d. Einstellen einer bestimmten Vergrößerungsstufe (halbautomatisch),
   e. Auswahl eines Bildausschnitts über Auswahirechteck im Übersichtsbild (halbautomatisch),
   f. Auswahl eines Bildausschnitts über Auswahlpunkt im Übersichtsbild (halbautomatisch) und/oder
   g. Auswahl einer Bildmarke (s. u.) (automatisch).
2. Eine Bilddokumentation über eine Markierungsebene umfasst die Optionen:
   h. Einfügen von Bildmarken (interaktiv),
   i. Einfügen von Text oder Symbolen (interaktiv),
   j. Einfügen von Grafiken oder freihändiges Einzeichnen (interaktiv) und/oder
   k. Einfügen von Verweisen oder Links (interaktiv).
3. Eine Intelligente Suche von Bildern oder Bildbereichen umfasst:
   I. Suche nach Markern (automatisch),
   m. Suche nach Textinhalten der Markierungsebene (automatisch),
   n. Suche nach Falldaten (automatisch) und/oder
   o. Suche nach Bildinhalten (Strukturen, Texturen, Formen, Farben) (halbautomatisch).
4. Daneben können folgende Messfunktionen vorgesehen sein:
   p. Automatisches Zählen von Strukturen (automatisch),
   q. Streckenberechnung mit Angabe der Strecke im reellen Maßstab (automatisch),
   r. Flächenberechnung mit Angabe der Fläche im reellen Maßstab (automatisch) und/oder
   s. Klassifikation von Texturen oder Farben (halbautomatisch).
5. Eine weitere Bildverarbeitungsfunktion kann sein:
   u. Vergleich mehrerer Bildlayer (Differenzbilder) (automatisch).
6. Weitere Funktionen können sein:
   v. Snapshot (Speichern von Teilbildern) (automatisch) und/oder
   w. Trackhistory (Registrierung des Betrachtungspfades) (automatisch).
7. Als Verwaltungsfunktionen können vorgesehen sein:
   x. Eingabe einer Beurteilung (interaktiv),
   y. Benutzerverwaltung (halbautomatisch),
   z. Falldatenverwaltung in der Datenbank (halbautomatisch) und/oder
   aa. Tracking eines Falles entlang des gesamten Workflows (automatisch).

### Oberfläche

Die Oberfläche läuft innerhalb eines Webbrowsers 111 oder eines unabhängigen Programmes. Das Programmfenster (Viewer) innerhalb des Browser- oder Programmfensters ist dabei vorzugsweise unterteilt in sechs Bereiche. Diese sechs Bereiche müssen nicht immer gleichzeitig erscheinen. Vielmehr kann der Anwender die von ihm gewünschten Oberflächenelemente selbst aktivieren, deaktivieren, und zusammenstellen.
1. Menüleiste
   Die Menüleiste enthält Steuerungen für alle Funktionen, die das Virtuelle-Mikroskop-System bietet. Zudem können über die Menüleiste die Verwaltung eines Falles gesteuert sowie alle Optionen und Systemattribute gesetzt und verändert werden.
2. Übersichtsbild
   Das Übersichtsbild zeigt anfangs das gesamte digitale Präparat (Virtueller Schnitt). Im Verlauf der Begutachtung dient es zur Auswahl einer neuen Bildansicht und zur Darstellung des aktuell angezeigten Bildbereiches. Hierzu erscheint im Übersichtsbild ein "Tracker", also ein Rechteck, dessen Begrenzungen mit den Rändern des Bildes der Bildansicht übereinstimmen. Mittels verschiedener Funktionen kann über den Tracker eine neue Bildansicht gewählt werden. Das Übersichtsbild selbst kann jedoch auch zur Anzeige einer Teilansicht veranlasst werden, wenn dies gewünscht ist.
3. Steuerungselemente
   Die Steuerungselemente umfassen verschiedene grafische Steuerungselemente, die die Navigation im Bild erleichtern sollen. Die Steuerung geschieht hier über die Maus. Alternativ kann auch über andere Eingabegeräte (Joystick, Touchpad, ...) oder über die Menüleiste navigiert werden.
4. Bildansicht
   Die Bildansicht zeigt das über das Übersichtsbild oder anderweitig ausgewählte aktuelle Teilbild. Anhand dieses Bildes kann der Anwender 13 ein Präparat begutachten. Das Übersichtsbild ist auf verschiedene Weisen navigierbar. Das kann über die Maus, über die Steuerungselemente, über das Übersichtsbild oder über andere Eingabegeräte geschehen. Zusätzlich können in der Bildansicht andere Bildlayer oder die Markierungsebene angezeigt werden, wahlweise auch in halbtransparenter Darstellung. Über die Maus ist ein Kontextmenü verfügbar, mit dem ortsgebundene Funktionen (beispielsweise das Setzen eines Markers) aktiviert werden können.
5. Statuszeile
   Die Statuszeile zeigt in einer Zeile Programmhinweise für den Anwender wie Progressbars, den Programmstatus etc.
6. Bildliste
   Die Bildliste ist eine Tabelle von kleinen Teilbildern, die entscheidende Inhalte des VS zusammenstellen, oder auch eine Tabelle von Übersichtsbildern aller VS eines Falles. Über Steuerungselemente ist es möglich, ein Bild auszuwählen, und in die Bildansicht umzuschalten.

### Eingabegeräte

Als Eingabegeräte werden Geräte bezeichnet, mit denen ein Anwender mit dem PC interagieren oder Daten eingeben kann. Diese Eingabe ist erforderlich bei Benutzerinteraktionen (Bedienung von Steuerungstools) oder bei der Dateneingabe. Die möglichen Eingabegeräte sind in der folgenden Tabelle zusammengestellt, wobei wiederum besonders bevorzugte Basisfunktionalitäten die Punkte 1 bis 4, vorteilhafte sinnvolle Hauptfunktionalitäten der Punkt 5 und optionale Funktionalitäten mit lediglich Zusatzfunktion die Punkte 6 bis 8 darstellen:
1. Bildaufnahmesystem
   Als System zur Digitalisierung der Bilddaten
2. Tastatur
   Zur Dateneingabe und Steuerung
3. 5-Tasten-Scrollmaus
4. Interface (z.B. HL7 oder DICOM-Scnittstelle)
   Zum Import von Daten aus anderen Datenbanksystemen
5. Joystick
   Werkzeug zur präzisen Bewegung eines Bildes. Dient auch zur Auswahl von Menüpunkten oder Ansteuerung von Buttons und ähnlichen Steuerungselementen.
6. Mikrofon
   Zur Dateneingabe (Aufsprechen hörbarer "Anmerkungen", In Verbindung mit einer Spracherkennungssoftware zur Eingabe von Textdaten) und Steuerung (Direkte Befehlseingabe mittels Spracherkennung, z.B. "Rauszoomen!", Menü- und Oberflächensteuerung mittels Spracherkennung, z.B. "Datei-Öffnen-Laufwerk A!").
7. Datenhandschuh (Handschuh mit Bewegungssensoren zur direkten Steuerung eines Mauszeigers/Bildes auf dem Bildschirm).
   Als Steuerungswerkzeug zum Bewegen von Bildern auf dem Bildschirm, Ansteuern von Menüs oder Buttons und ähnlicher Steuerungselemente oder auch für Bewegung in 3D-Objekten.
8. Gestenerkennung (Kamera mit Software zur Erkennung von Gesten und Handbewegungen. Denkbar auch Erkennung der Blickrichtung).
   Als Steuerungswerkzeug zum Bewegen von Bildern auf dem Bildschirm durch Bewegen der Hand oder bloßes Hinsehen, Ansteuern von Menüs oder Buttons und ähnlicher Steuerungselemente auf dem Bildschirm durch Zeigen oder bloßes Hinsehen.

Weitere einsetzbare, optionale Eingabegeräte sind Trackball, Touchscreen und/oder Touchpad:

### Ausgabegeräte

Unter Ausgabegeräten werden Geräte verstanden, mit denen ein Anwender Daten des Rechners wahrnehmen kann. Dazu zählen auch Geräte, mit denen ein entfemter Dritter die Daten wahrnehmen kann. Die möglichen Ausgabegeräte sind in der folgenden Tabelle zusammengestellt, wobei wieder besonders bevorzugte Basisfunktionalitäten die Punkte 1 bis 3, vorteilhafte sinnvolle Hauptfunktionalitäten die Punkte 4 und 5 und optionale Funktionalitäten mit lediglich Zusatzfunktion durch den Punkt 6 dargestellt sind:
1. Monitor (auch Touchscreen).
2. Modem bzw. Netzwerkkarte
   Zum Versenden eines Falles oder Teilen davon (z.B. einzelne Bilder) an entfernte Anwender durch Fax, E-Mail oder Direktverbindung (TCP/IP).
3. Interface (z.B. HL7 oder DICOM-Schnittstelle)
   Zum Export von Daten in andere Datenbanksysteme.
4. Lautsprecher
   Zur Ausgabe aufgesprochener Bemerkungen und zur Kommunikation bei Live-Diskussionen.
5. Drucker.
6. Beamer & Projektor.

### BEZUGSZEICHENLISTE

- 1: Clientsystem
11 Anwendersoftware
111 Webbrowser oder unabhängige Software-Oberfläche
12 Speichersystem
121 Zugangsdaten
13 Anwender
14 Zugriffssoftware auf externe Datenbanken
15 TCP/IP-Schnittstelle
- 2: Serversystem
21 Speichersystem
211 Zugangsdaten
212 Virtueller Schnitt
22 Datenbanken
221 Benutzerdatenbank
222 Falldatenbank
23 Serversoftware
231 Software zur Erzeugung Virtueller Schnitte
232 Software zur Bildbearbeitung Virtueller Schnitte
24 Externe Software
25 TCP/IP-Schnittstelle
- 3: Eingabedaten
31 digitale Bilddaten eines Präparates
32 Falldaten eines Präparates
- 4: Externe Datenbanken

## Patentansprüche

1. Virtuelles-Mikroskop-System mit
a. von einem Bildaufnahmesystem bereitgestellten oder anderweitig importierten digitalen Bilddaten (31) eines Präparates in der höchsten verfügbaren oder gewünschten Vergrößerung und dem höchsten verfügbaren oder gewünschten Ausschnitt des Präparates,
b. einem Serversystem (2) mit
- einer Software (231) zur Erzeugung eines Virtuellen Schnittes (212) in der höchsten verfügbaren oder gewünschten Vergrößerung und dem höchsten verfügbaren oder gewünschten Ausschnitt des Präparates aus den digitalen Bilddaten (31) des Präparates,
- einer Software (232) zur Bildbearbeitung des Virtuellen Schnittes (212) und
- einer Speichereinrichtung (21) zur Speicherung des Virtuellen Schnittes (212) in einer Falldatenbank (222), und
c. mindestens einem Clientsystem (1) mit einer Anwendersoftware (11) zur Darstellung von durch einen Anwender (13) auswählbaren Daten eines auswählbaren Virtuellen Schnittes (212), wobei ein durch den Anwender (13) ausgewählter Bildbereich des ausgewählten Virtuellen Schnittes (212) jeweils aktuell angefordert und dargestellt wird,
**dadurch gekennzeichnet, dass**
alle Daten jeweils eines Präparates als ein Virtueller Schnitt (212) in einer Datei abgelegt sind und dass clientseitig stets auf diese Datei des Virtuellen Schnitts (212) der höchsten verfügbaren oder gewünschten Vergrößerung und dem höchsten verfügbaren oder gewünschten Ausschnitt zugegriffen wird und
der Virtuelle Schnitt (212) eines Präparates ein Multilayer-Bild aus mehreren, einander überlagernden Bildlayern desselben Präparates umfasst, die durch automatisches Matching auf einen gemeinsamen geometrischen Bezug gebracht sind.

2. Virtuelles-Mikroskop-System nach Anspruch 1, **dadurch gekennzeichnet, dass** der höchste verfügbare oder gewünschte Ausschnitt des Präparates eine Gesamtansicht des Präparates ist.

3. Virtuelles-Mikroskop-System nach Anspruch 1 oder 2, **dadurch gekennzeichnet, dass** die Software (231) zur Erzeugung Virtueller Schnitte (212) eine Funktion zur Umformatierung der von dem Bildaufnahmesystem bereitgestellten digitalen Bilddaten (31) in ein komprimiertes Bildformat umfasst und die Virtuellen Schnitte (212) in dem komprimierten Bildformat in der Falldatenbank (222) vorliegen, insbesondere in Enhanced-Compressed-Wavelet-Format (ECW).

4. Virtuelles-Mikroskop-System nach einem der vorhergehenden Ansprüche, **dadurch gekennzeichnet, dass** die Anwendersoftware (11) des mindestens einen Clientsystems (1) Mittel umfasst, mit denen genau ein durch den Anwender (13) ausgewählter Bildbereich des ausgewählten Virtuellen Schnittes (212) in einer einer ausgewählten Auflösung entsprechenden Kompressionsstufe von der Falldatenbank (222) des Serversystems (2) jeweils aktuell angefordert oder von einem mobilen Datenträger geladen wird und dargestellt wird.

5. Virtuelles-Mikroskop-System nach einem der vorhergehenden Ansprüche, **dadurch gekennzeichnet, dass** die Anwendersoftware (11) einen Viewer umfasst, in dem die Gesamtansicht des Präparates und/oder der durch den Anwender (13) aktuell ausgewählte Bildbereich als Detailansicht darstellbar ist.

6. Virtuelles-Mikroskop-System nach einem der vorhergehenden Ansprüche, **dadurch gekennzeichnet, dass** die Anwendersoftware (11) eine stufenlose digitale Zoomfunktion umfasst, mit der eine Detailansicht eines beliebigen, von dem Anwender ausgewählten Ortes innerhalb der Gesamtansicht des Virtuellen Schnittes (212) bis zur höchsten Auflösung darstellbar ist und wobei das Zoomen durch interaktive Auswahl des Bildbereichs und Bestimmung einer Darstellungsgröße durch den Anwender (13) stufenlos durchführbar ist.

7. Virtuelles-Mikroskop-System nach Anspruch 5 oder 6, **dadurch gekennzeichnet, dass** die Anwendersoftware (11) einen in der Gesamtansicht des Präparates dargestellten Tracker umfasst, der die Position und Ausdehnung der dargestellten Detailansicht verdeutlicht und der durch den Anwender (13) zur Auswahl des Bildbereichs und zum manuellen Durchrastern des Virtuellen Schnittes (212) manuell setzbar, verschiebbar und dimensionierbar ist, wodurch eine neue Detailansicht gewählt wird.

8. Virtuelles-Mikroskop-System nach einem der vorhergehenden Ansprüche, **dadurch gekennzeichnet, dass** die von dem Aufnahmesystem übermittelten digitalen Bilddaten (31) in einem beliebigen Bilddatenformat vorliegen und durch die Software (231) zur Erzeugung der Virtuellen Schnitte (212) importierbar sind, wobei die Farbtiefe variabel festlegbar ist.

9. Virtuelles-Mikroskop-System nach einem der vorhergehenden Ansprüche, **dadurch gekennzeichnet, dass** die in der Falldatenbank (222) gespeicherten Virtuellen Schnitte (212) neben den Bilddaten weitere, durch einen Anwender (13) des Aufnahmesystems und/oder eines Clientsystems (1) eingebbare Daten (32), insbesondere Statusdaten des Falles, Aufnahmeparameter, Präparatdaten umfassen.

10. Virtuelles-Mikroskop-System nach Anspruch 9 **dadurch gekennzeichnet, dass** alle Daten der in der Falldatenbank (222) gespeicherten Virtuellen Schnitte (212) strukturiert, insbesondere SQL-strukturiert vorliegen.

11. Virtuelles-Mikroskop-System nach einem der vorhergehenden Ansprüche, **dadurch gekennzeichnet, dass** die Software (232) zur Bearbeitung der Virtuellen Schnitte (212) Bildbearbeitungsfunktionen umfasst, insbesondere Kontrastverbesserung; Normierung der Helligkeitsverteilung; Weißabgleich; Kantenextraktion; Matching auf bekannte Strukturen zur Vorauswahl interessierender Bereiche; Erkennen, Messen und Zählen von Objekten und Strukturen

12. Virtuelles-Mikroskop-System nach einem der vorhergehenden Ansprüche, **dadurch gekennzeichnet, dass** die Software (232) zur Bearbeitung der Virtuellen Schnitte (212) als implementierte Bildbearbeitungsfunktion eine Simulation von Beleuchtungssituationen aufweist, insbesondere eine Simulation wenigstens eines Hellfeldes und/oder Dunkelfeldes und/oder Polarisation und/oder Auflicht und/oder Durchlicht und/oder Phasenkontrast.

13. Virtuelles-Mikroskop-System nach einem der vorhergehenden Ansprüche, **dadurch gekennzeichnet, dass** die Software (232) zur Bearbeitung der Virtuellen Schnitte (212) als implementierte Bildbearbeitungsfunktion eine Simulation von Farbsituationen aufweist, insbesondere Erzeugung von Mischfärbungen als Kombination von realen Färbungen und/oder Farbwechsel.

14. Virtuelles-Mikroskop-System nach einem der vorhergehenden Ansprüche, **dadurch gekennzeichnet, dass** eine oder mehrere Bildbearbeitungsfunktionen der Software (232) nach Erzeugung des Virtuellen Schnittes (212) automatisch in Abhängigkeit eines Falltyps oder individuell durch einen Anwender (13) ausführbar sind.

15. Virtuelles-Mikroskop-System nach einem der vorhergehenden Ansprüche, **dadurch gekennzeichnet, dass** ein Konferenzmodus zwischen den Clientsystemen (1) vorgesehen ist.

16. Virtuelles-Mikroskop-System nach Anspruch 15, **dadurch gekennzeichnet, dass** zwischen den Clientsystemen (1) und/oder zwischen dem Clientsystem (1) und dem Serversystem (2) ein Steuerungswechsel, insbesondere eine Master-Slave-Zuordnung, vorgesehen ist.

17. Virtuelles-Mikroskop-System nach einem der vorhergehenden Ansprüche, **dadurch gekennzeichnet, dass** das Serversystem (2) Mittel (211, 221) zur Zugangssteuerung von Anwendern (13) auf das Serversystem (2) umfasst.

18. Virtuelles-Mikroskop-System nach Anspruch 17, **dadurch gekennzeichnet, dass** die Mittel (211, 221) zur Zugangssteuerung eine Benutzerdatenbank (221) mit hierin gespeicherten Anwenderdaten, Zugriffsrechten und Zuordnungen zu Fällen und dergleichen sowie Zugangsdaten (211) umfassen.

19. Virtuelles-Mikroskop-System nach Anspruch 17 oder 18, **dadurch gekennzeichnet, dass** die Zugangssteuerung Mittel zur Verschlüsselung/Entschlüsselung und/oder Mittel zur Prüfung der Zugangsberechtigung (digitale Signatur) umfasst.

20. Virtuelles-Mikroskop-System nach einem der vorhergehenden Ansprüche, **dadurch gekennzeichnet, dass** das mindestens eine Clientsystem (1) eine Client-Schnittstelle (15) und das Serversystem (2) eine Server-Schnittstelle (25) umfasst, die insbesondere TCP/IP-Schnittstellen sind, und eine Kommunikation zwischen Clientsystem (1) und Serversystem (2) über die Schnittstellen (15, 25) via Intranet oder Internet durchführbar ist, wobei auch mehrere Clientsysteme (1) gleichzeitig auf das Serversystem (2) zugreifen können.

21. Virtuelles Mikroskop nach einem der vorhergehenden Ansprüche, **dadurch gekennzeichnet, dass** ein Clientsystem (1) mit dem Serversystem (2) gemeinsam auf einer Rechnerstation installiert ist.

22. Virtuelles-Mikroskop-System nach einem der Ansprüche 1 bis 21, **dadurch gekennzeichnet, dass** die Anwendersoftware (11) innerhalb eines Webbrowsers (111) des Clientsystems (1) durch das Serversystem (2) bereitgestellt wird.

23. Virtuelles-Mikroskop-System nach einem der Ansprüche 1 bis 21, **dadurch gekennzeichnet, dass** die Anwendersoftware (11) als eigenständige Programmoberfläche im Clientsystem (1) oder innerhalb einer Programmoberfläche einer anderen Software im Clientsystem (1) bereitgestellt wird.

24. Virtuelles-Mikroskop-System nach einem der vorhergehenden Ansprüche, **dadurch gekennzeichnet, dass** im Serversystem (2) Mittel zur Zuordnung von Daten der Virtuellen Schnitte (212) zu Anwendern (13), Fallstatus, Falldaten und der Daten der Virtuellen Schnitte (212) untereinander vorgesehen sind.

25. Virtuelles-Mikroskop-System nach einem der vorhergehenden Ansprüche, **dadurch gekennzeichnet, dass** das Serversystem (2) und/oder das mindestens eine Clientsystem (1) Mittel zur Erzeugung von Multilayer-Bildern aus mehreren überlagernden Virtuellen Schnitten (212) desselben Präparates, gemessen unter Verwendung verschiedener Färbungen und/oder Aufnahmetechniken, umfasst.

26. Virtuelles-Mikroskop-System nach einem der vorhergehenden Ansprüchen, **dadurch kennzeichnet, dass** ein Zusammensetzen mehrerer Teilbilder zu einem Gesamtbild durch automatisches geometrisches Matching unterstützt wird.

27. Virtuelles-Mikroskop-System nach einem der vorhergehenden Ansprüchen, **dadurch gekennzeichnet, dass** durch Teilung einer Detailansicht, Umschalten der Detailansicht oder durch halbtransparente Darstellung, wobei das Maß der Transparenz einstellbar ist, mehrere Layer gleichzeitig darstellbar sind.

28. Virtuelles-Mikroskop-System nach Anspruch 27, **dadurch gekennzeichnet, dass** mehrere Detailansichten gleichzeitig darstellbar sind.

29. Virtuelles-Mikroskop-System nach Anspruch 28, **dadurch gekennzeichnet, dass** die Detailansichten in verschiedenen Vergrößerungen und/oder verschiedenen Färbungen und/oder verschiedenen Orten und/oder verschiedenen Zoomdarstellungen darstellbar sind.

30. Virtuelles-Mikroskop-System nach einem der vorhergehenden Ansprüche, **dadurch gekennzeichnet, dass** das Serversystem (2) und/oder das mindestens eine Clientsystem (1) Mittel zur Erzeugung einer mit den Bilddaten des Virtuellen Schnittes (212) überlagernden Markierungsebene umfasst, auf welcher durch den Anwender (13) Bildkoordinaten-bezogene Kommentare, Markierungen anbringbar und integriert in dem Virtuellen Schnitt (212) in der Falldatenbank speicherbar sind.

31. Virtuelles-Mikroskop-System nach Anspruch 30, **dadurch gekennzeichnet, dass** die Markierungsebene eine Markierung der während einer Analyse eines Virtuellen Schnittes (212) durch einen Anwender (13) betrachteten Bildbereiche und/oder eines Inspektionspfades umfasst, der einen von dem Anwender (13) inspizierten Weg durch das Präparat darstellt.

32. Virtuelles-Mikroskop-System nach einem der vorhergehenden Ansprüche, **dadurch gekennzeichnet, dass** ein Virtueller Schnitt (212) abrufbare Trackingdaten umfasst, die den Virtuellen Schnitt (212) betreffende und entlang des gesamten Workflows ständig protokollierte Vorgänge und Informationen enthalten, insbesondere den aktuellen Status, Anwender (13), Vorgang.

33. Virtuelles-Mikroskop-System nach einem der vorhergehenden Ansprüche, **dadurch gekennzeichnet, dass** die von dem Bildaufnahmesystem bereitgestellten digitalen Bilddaten (31) des Präparates und der serverseitig erzeugte Virtuelle Schnitt (212) ein aus Teilbereichen zusammengesetztes Bild des Präparates ist.

34. Virtuelles-Mikroskop-System nach einem der vorhergehenden Ansprüche, **dadurch gekennzeichnet, dass** die Bilddaten (31) digitale Schnitte, importierte Digitalbilder, PACS d.h. Picture Archivy and Communication System-Bilder, Livebilder, d.h. fernsteuerbare Mikroskope mit Kamera sind.

35. Verfahren zur Verarbeitung digitaler Mikroskopdaten, wobei
a. digitale Bilddaten (31) eines Präparates in der höchsten verfügbaren oder gewünschten Vergrößerung und dem höchsten verfügbaren oder gewünschten Ausschnitt des Präparates von einem Bildaufnahmesystem bereitgestellt oder anderweitig importiert werden,
b. mittels einer ersten Software (231) eines Serversystems (2) aus den digitalen Bilddaten (31) des Präparates ein Virtueller Schnitt (212) in der höchsten verfügbaren oder gewünschten Vergrößerung und dem höchsten verfügbaren oder gewünschten Ausschnitt erzeugt wird,
c. mittels einer zweiten Software (232) des Serversystems (2) der Virtuelle Schnitt (212) mittels Bildbearbeitungsfunktionen verändert wird,
d. der Virtuelle Schnitt (212) in einer Falldatenbank (222) einer Speichereinrichtung (21) gespeichert wird und
e. mittels einer Anwendersoftware (11) mindestens eines Clientsystems (1) durch einen Anwender (13) ausgewählte Daten eines ausgewählten Virtuellen Schnittes (212) der Falldatenbank (222) dargestellt werden, wobei ein durch den Anwender (13) ausgewählter Bildbereich des ausgewählten Virtuellen Schnittes (212) jeweils aktuell angefordert und dargestellt wird,
**dadurch gekennzeichnet, dass**
alle Daten jeweils eines Präparates als ein Virtueller Schnitt (212) in einer Datei abgelegt werden und clientseitig stets auf diese Datei des Virtuellen Schnitts (212) der höchsten verfügbaren oder gewünschten Vergrößerung und dem höchsten verfügbaren oder gewünschten Ausschnitt des Präparates zugegriffen wird und
der Virtuelle Schnitt (212) eines Präparates ein Multilayer-Bild aus mehreren, einander, überlagernden Bild-layern desselben Präparates umfasst, die durch automatisches Matching auf einen gemeinsamen geometrischen Bezug gebracht sind.

36. Verfahren nach Anspruch 35, **dadurch gekennzeichnet, dass** der höchste verfügbare oder gewünschte Ausschnitt des Präparates eine Gesamtansicht des Präparates ist.

37. Verfahren nach Anspruch 35 oder 36, **dadurch gekennzeichnet,** mittels der ersten Software (231) zur Erzeugung Virtueller Schnitte (212) die von dem BildaufnahmeSystem bereitgestellten digitalen Bilddaten (31) in ein komprimiertes Bildformat umformatiert werden, insbesondere in Enhanced-Compressed-Wavelet-Format (ECW), und die Virtuellen Schnitte (212) in dem komprimierten Bildformat in der Falldatenbank (222) gespeichert werden.

## Claims

1. Virtual-microscope system with
a. digital-image data (31) of a specimen provided by an image-recording system or imported from elsewhere in the maximum available or desired
magnification and the maximum available or desired detail of the specimen, b. a server system (2) with
- a software program (231) for generating a virtual section (212) in the maximum available or desired magnification and the maximum available or desired detail of the specimen from the digital-image data (31) of the specimen;
- a software program (232) for image processing of the virtual section (212); and
- a memory device (21) for storage of the virtual section (212) in a case databank (222); and
c. at least one client system (1) with a user software program (11) for the presentation of data of a selectable virtual section (212) selectable by a user (13), wherein an image region of the selected virtual section (212) selected by the user (13) is currently requested and presented respectively,
**characterised in that**
all of the data of a respective specimen are stored as a virtual section (212) in one file, and the client always gains access to this file of the virtual section (212) of the maximum available or desired magnification and the maximum available or desired detail of the specimen, and that the virtual section (212) of a specimen comprises a multi-layered image composed of several image layers of the same specimen overlaid one over the other, which are brought by automatic matching into a common geometric relationship.

2. Virtual-microscope system according to claim 1, **characterised in that** the maximum available or desired detail of the specimen is a total view of the specimen.

3. Virtual-microscope system according to claim 1 or 2, **characterised in that** the software (231) for generating virtual sections (212) comprises a function for re-formatting the digital-image data (31) provided by the image-recording system into a compressed-image format, and the virtual sections (212) are present in the case databank (222) in the compressed-image format, especially in the enhanced-compressed-wavelet-format (ECW).

4. Virtual-microscope system according to any one of the preceding claims,
**characterised in that**
the user software (11) of the at least one client system (1) comprises means, with which precisely one image region of the selected virtual section (212) selected by the user (13) is currently requested in a compression stage corresponding to a selected resolution from the case databank (222) of the server system (2) or loaded from a mobile data medium and presented respectively.

5. Virtual-microscope system according to any one of the preceding claims, **characterised in that** the user software (11) comprises a viewer, in which the total view of the specimen and/or the image region currently selected by the user (13) can be presented as a detail view.

6. Virtual-microscope system according to any one of the preceding claims, **characterised in that** the user software (11) comprises an infinitely-adjustable digital zoom function, with which a detail view of any position selected by the user within the total view of the virtual section (212) can be presented up to the maximum resolution and wherein the zoom function can be implemented in an infinitely-adjustable manner by interactive selection of the image region and determination of a presentation size by the user (13).

7. Virtual-microscope system according to claim 5 or 6, **characterised in that** the user software (11) comprises a tracker presented in the total view of the specimen, which explains the position and extension of the detail view presented and which can be manually set, displaced and dimensioned by the user (13) in order to select the image region and manually screen through the virtual section (212), thereby selecting a new detail view.

8. Virtual-microscope system according to any one of the preceding claims, **characterised in that** the digital-image data (31) communicated from the recording system are present in any image-data format and can be imported by the software program (231) for generating the virtual sections (212), wherein the depth of colour can be set in a variable manner.

9. Virtual-microscope system according to any one of the preceding claims, **characterised in that** the virtual sections (212) stored in the case databank (222) comprise, alongside the image data, other data (32), which can be entered by a user (13) of the recording system and/or of a client system, in particular, status data of the case, recording parameters and specimen data.

10. Virtual-microscope system according to claim 9, **characterised in that** all of the data of the virtual sections (212) stored in the case databank (222) are present in a structured format, especially in an SQL structured format.

11. Virtual-microscope system according to any one of the preceding claims, **characterised in that** the software program (232) for processing the virtual sections (212) comprises image-processing functions, especially contrast enhancement; scaling of brightness distribution; white balance; edge extraction; matching to known structures to allow pre-selection of relevant regions; detection, measurement and counting of objects and structures.

12. Virtual-microscope system according to any one of the preceding claims, **characterised in that** the software program (232) for processing the virtual sections (212) provides, as an implemented image-processing function, a simulation of lighting situations, especially a simulation of at least one bright field and/or dark field and/or polarisation and/or incident light and/or transmitted light and/or phase contrast.

13. Virtual-microscope system according to any one of the preceding claims, **characterised in that** the software program (232) for processing the virtual sections (212) provides, as an implemented image-processing function, a simulation of colour situations, especially a generation of mixed stainings as a combination of real stainings and/or a colour change.

14. Virtual-microscope system according to any one of the preceding claims, **characterised in that** one or more image-processing functions of the software program (232) can be implemented after the generation of the virtual section (212) either automatically dependent upon a case type or individually by a user (13).

15. Virtual-microscope system according to any one of the preceding claims, **characterised in that** a conference mode between the client systems (1) is provided.

16. Virtual-microscope system according to claim 15, **characterised in that** a control change, especially a master-slave assignment, is provided between the client systems (1) and/or between the client system (1) and the server system (2).

17. Virtual-microscope system according to any one of the preceding claims, **characterised in that** the server system (2) comprises means (211, 221) for controlling user (13) access to the server system (2).

18. Virtual-microscope system according to claim 17, **characterised in that** the means (211, 221) for access control comprises a user databank (221), in which user data, access rights and assignments to cases and similar are stored together with access data (211).

19. Virtual-microscope system according to claim 17 or 18, **characterised in that** the access control comprises means for coding/decoding and/or means for testing the access authorisation (digital signature).

20. Virtual-microscope system according to any one of the preceding claims, **characterised in that** the at least one client system (1) comprises a client interface (15), and the server system (2) comprises a server interface (25), which are, in particular, TCP/IP interfaces, and that communication can be implemented between client system (1) and server system (2) through the interfaces (15, 25) via intranet or internet, wherein several client systems (1) can also have simultaneous access to the server system (2).

21. Virtual microscope according to any one of the preceding claims, **characterised in that** a client system (1) is installed together with the server system (2) in one computer station.

22. Virtual-microscope system according to any one of claims 1 to 21, **characterised in that** the user software (11) is provided by the server system (2) within a web browser (111) of the client system (1).

23. Virtual-microscope system according to any one of claims 1 to 21, **characterised in that** the user software (11) is provided as an independent program surface in the client system (1) or within a program surface of another software program in the client system (1).

24. Virtual-microscope system according to any one of the preceding claims, **characterised in that**, means are provided within the server system (2) for assigning data of the virtual sections (212) to users (13) and for assigning case status, case data and data of the virtual sections (212) to one another.

25. Virtual-microscope system according to any one of the preceding claims, **characterised in that** the server system (2) and/or the at least one client system (1) comprises means for generating multi-layered images composed of several overlaid virtual sections (212) of the same specimen, measured using different stainings and/or recording techniques.

26. Virtual-microscope system according to any one of the preceding claims, **characterised in that** a combination of several individual images to form a total image through automatic geometric matching is supported.

27. Virtual-microscope system according to any one of the preceding claims, **characterised in that** several layers can be presented simultaneously by dividing a detail view, by switching the detail view or by semi-transparent presentation, wherein the degree of transparency is adjustable.

28. Virtual-microscope system according to claim 27, **characterised in that** several detail views can be presented simultaneously.

29. Virtual-microscope system according to claim 28, **characterised in that** the detail views can be presented in the various magnifications and/or various stainings and/or various positions and/or various zoom presentations.

30. Virtual-microscope system according to any one of the preceding claims, **characterised in that** the server system (2) and/or the at least one client system (1) comprises means for generating a marking plane overlaid with the image data of the virtual section (212), to which comments relating to the image-coordinates and markings can be attached by the user (13) and stored in the case databank integrated with the virtual section (212).

31. Virtual-microscope system according to claim 30, **characterised in that** the marking plane comprises a marking of the image regions observed by a user (13) during an analysis of a virtual section (212) and/or of an inspection path, which represents a route through the specimen inspected by the user (13).

32. Virtual-microscope system according to any one of the preceding claims, **characterised in that** a virtual section (212) comprises retrievable tracking data, which contain processes and information relating to the virtual section (212) and continuously logged throughout the workflow, especially the current status, the user (13) and the procedure.

33. Virtual-microscope system according to any one of the preceding claims, **characterised in that** the digital-image data (31) of the specimen provided
by the image-recording system and the virtual section (212) generated by the server is an image of the specimen composed of individual regions.

34. Virtual-microscope system according to any one of the preceding claims, **characterised in that** the image data (31) are digital sections, imported digital images, PACS, i.e. Picture Archive and Communication System images, live-images, i.e. remote-controllable microscopes with camera, and the like.

35. Method for processing digital-microscope data, wherein
a. digital-image data (31) of a specimen are provided by an image-recording system or imported from elsewhere in the maximum available or desired
magnification and the maximum available or desired detail of the specimen; b. a virtual section (212) is generated from the image data (31) of the specimen in the maximum available or desired magnification and the maximum available or desired detail by means of a first software program (231) of a server system (2);
c. the virtual section (212) is modified by means of a second software program (232) of the server system (2) using image-processing functions;
d. the virtual section (212) is stored in a case databank (222) of a memory device (21) and
e. data of a selected virtual section (212) of the case databank (222) selected by a user (13) are presented by means of a user software program (11) of at least one client system (1), wherein an image region of the selected virtual section (212) selected by the user (13) is currently requested and presented respectively,
**characterised in that**
all of the data of one respective specimen are stored as a virtual section (212) in one file, and the client always gains access to this file of the virtual section (212) of the maximum available or desired magnification and the maximum available or desired detail of the specimen and
the virtual section (212) of a specimen comprises a multi-layered image composed of several image layers of the same specimen overlaid one over the other, which are brought by automatic matching into a common geometric relationship.

36. Method according to claim 35, **characterised in that** the maximum available or desired detail of the specimen is a total view of the specimen.

37. Method according to claim 35 or 36, **characterised in that** the digital-image data (31) provided by the image recording system are re-formatted by means of the first software program (231) for generating virtual sections (212) into a compressed-image format, especially into the enhanced-compressed-wavelet format (ECW), and the virtual sections (212) are stored in the case databank (222) in the compressed-image format.

## Revendications

1. Système de microscope virtuel comprenant
a. des données d'images numériques (31) d'une préparation, mises à disposition à partir d'un système de prise de vues ou importées par ailleurs, dans l'agrandissement le plus élevé disponible ou souhaité et dans la section la plus disponible ou souhaitée de la préparation,
b. un système de serveur (2) avec
- un logiciel (231) en vue de la production d'une coupe virtuelle (212) dans l'agrandissement le plus élevé disponible ou souhaité et dans la section la plus disponible ou souhaitée de la préparation, obtenue à partir des données d'image numériques (31) de la préparation,
- un logiciel (232) en vue du traitement d'images de la coupe virtuelle (212) et
- un équipement de sauvegarde (21) en vue de la sauvegarde de la coupe virtuelle (212) dans une banque de données de cas (222), et
c. au moins un système client (1) avec un logiciel utilisateur (11), en vue de la représentation d'une coupe virtuelle sélectionnable (212) par des données sélectionnables par un utilisateur (13), par lequel un domaine d'image sélectionnable par l'utilisateur (13) de la coupe virtuelle sélectionnable (212) est, à chaque fois, en temps actuel demandé et représenté,
**caractérisé en ce que** toutes les données respectivement d'une préparation sont placées en tant qu'une coupe virtuelle (212) dans un fichier et **en ce que**, côté client, on a accès à ce fichier de la coupe virtuelle (212) dans l'agrandissement le plus élevé disponible ou souhaité et dans la section la plus disponible ou souhaitée et **en ce que** la coupe virtuelle (212) d'une préparation comprend une image multicouches faites de plusieurs couches d'image, superposées les unes aux autres, qui sont amenées par un assortiment automatique sur une référence géométrique commune.

2. Système de microscope virtuel selon la revendication 1, **caractérisé en ce que** la section la plus disponible ou souhaitée de la préparation est une vue d'ensemble de la préparation.

3. Système de microscope virtuel selon la revendication 1 ou 2, **caractérisé en ce que** le logiciel (231) comprend, en vue de la production de coupes virtuelles (212), une fonction en vue du reformatage des données d'image numériques mises à disposition par le système de prises de vue (31) sous la forme d'un format d'images comprimé et **en ce que** les coupes virtuelles (212) sont présentes sous la forme d'un format d'images comprimé dans la banque de données de cas (222), en particulier sous la forme d'un format Enhanced-Compressed-Wavelet (ECW).

4. Système de microscope virtuel selon l'une quelconque des revendications précédentes, **caractérisé en ce que** le logiciel utilisateur (11) du au moins un système client (1) comprend des moyens, grâce auxquels un domaine d'images sélectionné par l'utilisateur (13) de la coupe virtuelle sélectionnée (212) est demandé à chaque fois en temps actuel dans une étape de compression correspondant à une résolution sélectionnée de la banque de données de cas (222) du système serveur (2) ou est chargé à partir d'un support de données mobile et est représenté.

5. Système de microscope virtuel selon l'une quelconque des revendications précédentes, **caractérisé en ce que** le logiciel pour utilisateur (11) comprend un dispositif de visualisation, dans lequel la vue d'ensemble de la préparation et/ou le domaine d'image sélectionnée actuellement par l'utilisateur (13) peut être représenté(e) en tant que vue de détails.

6. Système de microscope virtuel selon l'une quelconque des revendications précédentes, **caractérisé en ce que** le logiciel utilisateur (11) comprend une fonction zoom numérique en mode continu, grâce à laquelle une vue de détails d'un endroit, sélectionné par l'utilisateur au sein de la vue d'ensemble de la coupe virtuelle (212), peut être représentée jusqu'à la résolution la plus élevée et moyennant quoi l'opération de zoom peut être effectuée en mode continu par l'utilisateur (13) par une sélection interactive du domaine d'images et par une détermination d'une taille de représentation.

7. Système de microscope virtuel selon la revendication 5 ou 6, **caractérisé en ce que** le logiciel pour utilisateurs (11) comprend un dispositif de suivi représenté dans la vue d'ensemble de la préparation, qui met en évidence la position et l'extension de la vue de détails représentée et qui peut être réglé, décalé et dimensionné à la main par l'utilisateur, en vue de la sélection du domaine d'images et en vue du cadrage manuel de la coupe virtuelle (212), à la suite de quoi une nouvelle vue de détails est sélectionnée.

8. Système de microscope virtuel selon l'une quelconque des revendications précédentes, **caractérisé en ce que** les données d'images numériques transmises par le système de prises de vue sont présentes sous la forme d'un format de données d'images quelconque et peuvent être importées par le logiciel (231) en vue de la production des coupes virtuelles (212), l'intensité de la coloration pouvant être fixée d'une manière variable.

9. Système de microscope virtuel selon l'une quelconque des revendications précédentes, **caractérisé en ce que** les coupes virtuelles (212), sauvegardées dans la banque de données de cas (222), comprennent, outre les données d'images, d'autres données saisissables par l'utilisateur (13) du système de prises de vue et/ou d'un système pour clients (1), en particulier les données de statut du cas, les paramètres de prise de vue, les données de la préparation.

10. Système de microscope virtuel selon la revendication 9, **caractérisé en ce que** toutes les données des coupes virtuelles (212) sauvegardées dans la banque de données de cas (222), sont présentes sous une forme structurée, en particulier sous une forme structurée SQL.

11. Système de microscope virtuel selon l'une quelconque des revendications précédentes, **caractérisé en ce que** le logiciel (232) comprend, en vue du traitement des coupes virtuelles (212), des fonctions de traitement d'images, en particulier une amélioration du contraste; une standardisation de la distribution de luminosité; une compensation du blanc; une extraction des bords; un calage sur des structures connues en vue de la présélection de domaines intéressants; une reconnaissance, une mesure et une comptabilisation d'objets et de structures.

12. Système de microscope virtuel selon l'une quelconque des revendications précédentes, **caractérisé en ce que** le logiciel (232) présente, en vue du traitement des coupes virtuelles (212) en tant que fonction de traitement d'image implémentée, une simulation de situations d'illumination, en particulier une simulation d'au moins un champ clair et/ou d'un champ sombre et/ou de polarisation et/ou de lumière incidente et/ou de lumière transmise et/ou de contraste de phases.

13. Système de microscope virtuel selon l'une quelconque des revendications précédentes, **caractérisé en ce que** le logiciel (232) présente, en vue du traitement des coupes virtuelles (212), en tant que fonction de traitement implémentée, une simulation de situations de coloration, en particulier la production de colorations mixtes en tant que combinaison de colorations réelles et/ou de changement de couleurs.

14. Système de microscope virtuel selon l'une quelconque des revendications précédentes, **caractérisé en ce qu'**une ou plusieurs fonctions de traitement d'images du logiciel (232), après production de la coupe virtuelle (212), peuvent être exécutées automatiquement en fonction d'un type de cas ou individuellement par l'utilisateur (13).

15. Système de microscope virtuel selon l'une quelconque des revendications précédentes, **caractérisé en ce que** l'on prévoit un mode conférence entre les systèmes clients (1).

16. Système de microscope virtuel selon la revendication 15, **caractérisé en ce qu'**entre les systèmes clients (1) et/ou entre le système client (1) et le système de serveur (2), l'on prévoit un échange de commande, en particulier une relation maître-esclave.

17. Système de microscope virtuel selon l'une quelconque des revendications précédentes, **caractérisé en ce que** le système serveur (2) comprend des moyens (211, 221) en vue de la commande d'accès des utilisateurs (13) au système serveur (2).

18. Système de microscope virtuel selon la revendication 17, **caractérisé en ce que** les moyens (211, 221) en vue de la commande d'accès d'une banque de données d'utilisateurs (221), ayant des données d'utilisateur qui y sont sauvegardées, comprennent des droits d'accès et des attributions aux cas et similaires ainsi que des données d'accès (211).

19. Système de microscope virtuel selon la revendication 17 ou 18, **caractérisé en ce que** la commande d'accès comprend des moyens en vue du chiffrement / déchiffrement des moyens en vue du contrôle de l'autorisation d'accès (signature numérique).

20. Système de microscope virtuel selon l'une quelconque des revendications précédentes, **caractérisé en ce que** le au moins un système client (1) comprend une interface client (15), **en ce que** le système de serveur (2) comprend une interface serveur (25), qui sont des interfaces TCP/IP et **en ce qu'**une communication entre le système client (1) et le système de serveur (2) peut être effectuée par l'intermédiaire des interfaces (15, 25) via Intranet ou Internet, plusieurs systèmes client (1) pouvant également simultanément accéder au système de serveur (2).

21. Système de microscope virtuel selon l'une quelconque des revendications précédentes, **caractérisé en ce qu'**un système client (1) peut être installé sur une station d'ordinateur conjointement au système serveur (2).

22. Système de microscope virtuel selon l'une quelconque des revendications 1 à 21, **caractérisé en ce que** le logiciel utilisateur (11) est mis à disposition par le système de serveur (2) au sein d'un navigateur WEB (111) du système client (1).

23. Système de microscope virtuel selon l'une quelconque des revendications 1 à 21, **caractérisé en ce que** le logiciel utilisateur (11) est mis à disposition en tant que couche de programme indépendante dans le système client (1) ou au sein d'une couche de programme d'un autre logiciel.

24. Système de microscope virtuel selon l'une quelconque des revendications précédentes, **caractérisé en ce que**, dans le système serveur (2), l'on prévoit des moyens en vue de la répartition de données des coupes virtuelles (212) aux utilisateurs (13), selon le statut de cas, les données de cas et les données des coupes virtuelles (212) entre elles.

25. Système de microscope virtuel selon l'une quelconque des revendications précédentes, **caractérisé en ce que** le système serveur (2) et/ou le au moins un système client (1) comprend des moyens en vue de la production d'images multicouches faites de plusieurs coupes virtuelles (212) superposées de la même préparation, évaluées sous utilisation de colorations et/ou de techniques de prise de vues variées.

26. Système de microscope virtuel selon l'une quelconque des revendications précédentes, **caractérisé en ce qu'**une combinaison de plusieurs images partielles pour former une image globale par assortiment géométrique automatique est supportée.

27. Système de microscope virtuel selon l'une quelconque des revendications précédentes, **caractérisé en ce qu'**il permet de représenter simultanément plusieurs couches par division d'une vue de détails, par commutation de la vue de détails ou par représentation semi-transparente, moyennant quoi l'amplitude de la transparence est réglable.

28. Système de microscope virtuel selon la revendication 27, **caractérisé en ce que** plusieurs vues de détails peuvent être représentées d'une manière simultanée.

29. Système de microscope virtuel selon la revendication 28, **caractérisé en ce que** les vues de détails peuvent être représentées dans des agrandissements divers et/ou des colorations diverses et/ou des endroits divers et/ou des représentations de zoom diverses.

30. Système de microscope virtuel selon l'une quelconque des revendications précédentes, **caractérisé en ce que** le système serveur (2) et/ou le au moins un système client (1) comprend des moyens en vue de la production d'un plan de marquage se superposant aux données d'images de la coupe virtuelle (212), sur lequel des commentaires, des marquages relatifs aux coordonnées d'images peuvent être apposées par l'utilisateur (13) et peuvent être sauvegardées d'une manière intégrée à la coupe virtuelle (212) dans la banque de données de cas.

31. Système de microscope virtuel selon la revendication 30, **caractérisé en ce que** le plan de marquage comprend un marquage des domaines d'images observés par un utilisateur (13) pendant l'analyse d'une coupe virtuelle (212) et/ou d'un trajet d'inspection, qui représente un trajet inspecté par l'utilisateur (13) à travers la préparation.

32. Système de microscope virtuel selon l'une quelconque des revendications précédentes, **caractérisé en ce qu'**une coupe virtuelle (212) comprend des données de suivi que l'on peut appeler, lesquelles contiennent les procédures et les informations concernant la coupe virtuelle (212) et enregistrées en permanence le long du tracé de travail tout entier, en particulier le statut actuel, l'utilisateur (13), la procédure.

33. Système de microscope virtuel selon l'une quelconque des revendications précédentes, **caractérisé en ce que** les données d'image numériques (31) de la préparation, mises à disposition par le système de prises de vue, et de la coupe virtuelle (212) produite côté serveur sont une image combinée à partir de domaines partiels de la préparation.

34. Système de microscope virtuel selon l'une quelconque des revendications précédentes, **caractérisé en ce que** les données d'images (31) sont des coupes numériques, des images numériques importées, des images PACS, c'est-à-dire Picture Archivy and Communication System, des images "live", c'est-à-dire obtenues par un microscope télécommandé avec appareil photo.

35. Procédé en vue du traitement de données de microscope numériques, dans lequel :
a. des données d'image numériques (31) d'une préparation sont mises à disposition par un système de prises de vue ou sont importées par ailleurs, dans l'agrandissement le plus élevé disponible ou souhaité et dans la section la plus disponible ou souhaitée de la préparation,
b. une coupe virtuelle (212) est produite, grâce à un premier logiciel (231) d'un système serveur (2) à partir de données d'images de la préparation, dans l'agrandissement le plus élevé disponible ou souhaité et dans la section la plus disponible ou souhaitée,
c. la coupe virtuelle (212) est modifiée à l'aide d'un deuxième logiciel (232) du système serveur (2) par l'intermédiaire de fonctions de traitement d'images,
d. la coupe virtuelle (212) est sauvegardée dans une banque de données de cas (222) d'un équipement de sauvegarde (21),
e. les données d'une coupe virtuelle (212) sélectionnée de la banque de données de cas (222), sélectionnée par l'utilisateur (13) sont représentées à l'aide d'un logiciel utilisateur (11) d'au moins un système client (1), un domaine d'images sélectionnée par l'utilisateur de la coupe virtuelle (212) sélectionné étant, respectivement, fourni en temps actuel et représenté,
**caractérisé en ce que** toutes les données respectives d'une préparation sont placées en tant qu'une coupe virtuelle (212) dans un fichier et **en ce que**, côté client, on a accès à ce fichier de la coupe virtuelle (212) dans l'agrandissement le plus élevé disponible ou souhaité et dans la section la plus disponible ou souhaitée et **en ce que** la coupe virtuelle (212) d'une préparation comprend une image multicouches faites de plusieurs couches d'images de la même préparation, superposées les unes aux autres, qui sont amenées par un calage automatique sur une référence géométrique commune.

36. Procédé selon la revendication 35, **caractérisé en ce que** la section la plus disponible ou souhaitée de la préparation est une vue d'ensemble de la préparation.

37. Procédé selon la revendication 35 ou 36, **caractérisé en ce que**, grâce au premier logiciel (231) en vue de la production de coupes virtuelles (212), les données d'images numériques mises à disposition par le système de prises de vue (31) sont reformatées sous la forme d'un format d'images comprimé, en particulier sous la forme d'un format Enhanced-Compressed-Wavelet (ECW) et **en ce que** les coupes virtuelles (212) sont sauvegardées sous la forme d'un format d'images comprimé dans la banque de données de cas (222).
